# EUROPEAN PATENT APPLICATION

(11) **EP 4 840 457 A1**
(43) Date of publication of application: **18.11.2026**
(21) Application number: 26176988.9
(22) Date of filing: 06.05.2026
(51) Int. Cl.: A61K 36/48, A61K 36/81, A61P 15/08, A61K 31/35, A61K 33/30

(54) **COMPOSITION COMPRISING FENUGREEK FOR USE IN THE TREATMENT OF FEMALE INFERTILITY**

(30) Priority: 16.05.2025 IT 202500011054
(71) Applicant: Androsystems- S.r.l., 00161 Roma (IT)
(72) Inventor: GRECO, Pierfrancesco, 00191 ROMA (IT)
(74) Representative: Modiano, Micaela Nadia

(57) **Abstract**

A composition comprising fenugreek for use in the treatment of female infertility and in particular for use in the improvement of the quality and quantity of ovarian reserve.

## Description

The present invention relates to a composition for use in the treatment of female infertility.

Fenugreek (Trigonella foenum-graecum) is a plant belonging to the legume family, which typically grows to a height of 60 cm. It has small green leaves and white or yellowish flowers that give way to small golden-brown seeds. These seeds are the most commonly used part of the plant, for both culinary and medicinal purposes. The seeds have a strong, distinctive odor reminiscent of maple syrup and a flavor that is often described as a combination of bitter and nutty.

Fenugreek seeds are rich in a wide range of nutrients, making them a healthy addition to any diet. Some of the key nutrients in fenugreek seeds include:
- Fiber: fenugreek is rich in soluble fiber, which helps regulate blood sugar levels and supports digestive system health.
- Vitamins: it contains significant amounts of vitamins such as vitamin A, vitamin C, and various B-group vitamins.
- Minerals: fenugreek seeds are rich in iron, magnesium, potassium, and calcium.
- Protein: fenugreek is a good source of plant-based protein, which supports muscle growth and repair.
- Phytochemicals: the seeds are rich in saponins, flavonoids, and alkaloids, which contribute to their medicinal properties.

Fenugreek has traditionally been used to treat a variety of health issues, from digestive problems to hormonal imbalances. Some of the most studied benefits include:

### Promoting lactation

One of the best-known uses of fenugreek is its ability to stimulate milk production in breastfeeding mothers. Several studies have shown that fenugreek can increase breast milk production, making it a popular herbal remedy for mothers struggling with low milk production. It is believed that the active compounds in fenugreek, such as diosgenin, mimic estrogen and help stimulate the milk-producing glands.

### Facilitates blood sugar control

Fenugreek has also garnered attention for its potential in regulating blood sugar levels. Several studies suggest that fenugreek seeds may help to improve insulin sensitivity, which is particularly beneficial for people with type 2 diabetes. Fenugreek's high fiber content slows the absorption of sugars, preventing blood sugar spikes.

### Reduces inflammation

Fenugreek contains several compounds with anti-inflammatory properties. These compounds may help to alleviate symptoms of conditions such as arthritis and other inflammatory disorders. Regular consumption of fenugreek may also help to improve joint health, reducing pain and swelling.

### Improves digestion

It is known that fenugreek seeds aid digestion by stimulating the production of gastric acids and promoting healthy bowel movements. The fiber contained in fenugreek helps prevent constipation, supports gut health, and reduces bloating. It is also useful in treating indigestion and gastroesophageal reflux.

### Boosts testosterone levels

Fenugreek is believed to have a positive effect on male hormone levels. Some studies suggest that fenugreek supplementation can increase testosterone levels, improve libido, and enhance sexual function in men. It may moreover support muscle strength and athletic performance, which is why it is sometimes used by bodybuilders and athletes.

### Contributes to heart health

Fenugreek's high fiber content and its ability to lower blood sugar levels also contribute to heart health. By helping to regulate blood sugar and cholesterol levels, fenugreek may reduce the risk of cardiovascular disease. Some studies suggest that fenugreek may help to reduce total cholesterol and LDL (bad) cholesterol, while increasing HDL (good) cholesterol.

In women, considering all published studies, fenugreek has been used as a galactagogue, for dysmenorrhea, during menopause to reduce vasomotor symptoms and depression, and to improve sexual function in healthy menstruating women. However, there are no reports in the literature of fenugreek being used to improve female fertility.

Female infertility is very often due to diminished ovarian reserve. Ovarian reserve is the term used to describe the number and quality of eggs in a woman's ovaries. Diminished ovarian reserve (DOR) refers to a condition in which the ovaries contain fewer eggs than expected for the woman's age. This condition can affect fertility, causing difficulty conceiving. With growing awareness of DOR, it is important to understand its nature, causes, and treatment options available to women facing this condition. As women age, their number of eggs naturally decreases, with a more significant reduction typically occurring after age 35. However, some women may experience a decline in egg quantity and quality at an early age.

DOR is diagnosed through blood tests that measure levels of specific hormones such as anti-Müllerian hormone (AMH), follicle-stimulating hormone (FSH), and estradiol, and through ultrasonograms to assess the count of antral follicles (AFC), small fluid-filled sacs in the ovaries which contain the eggs.

Although there is no established definition of DOR, the Bologna criteria, recently reported by the ESHRE working group (Ferraretti AP, La Marca A, Fauser BC, Tarlatzis B, Nargund G, Gianaroli L; ESHRE working group on Poor Ovarian Response Definition. ESHRE consensus on the definition of 'poor response' to ovarian stimulation for in vitro fertilization: the Bologna criteria. Hum Reprod. 2011 Jul;26 (7):1616-24), consider a follicle count <5 and an AMH value <1.2 ng/ml.

The most common cause of diminished ovarian reserve in women is age, but it may also be due to genetic factors, medical conditions, previous ovarian surgery, and lifestyle factors.

Diminished ovarian reserve is irreversible, and the treatment options currently used to assist women in their fertility journey are:
- ovarian stimulation and in vitro fertilization (IVF);
- preventive oocyte freezing;
- lifestyle modifications;
- ovarian donation.

A new treatment option for women with diminished ovarian reserve is therapy with platelet-rich plasma (PRP). This treatment involves drawing a blood sample from the woman, processing it to concentrate the platelets, and injecting this PRP directly into the ovaries. It is believed that the growth factors and proteins present in PRP stimulate ovarian tissue, potentially improving oocyte quality and ovarian function.

Although this treatment is still relatively new and experimental, some women with diminished ovarian reserve have reported improvements in ovarian function following PRP therapy. However, further research is needed to confirm its long-term effectiveness. Therefore, there is actually no cure to improve diminished ovarian reserve. The various treatment options can only help women with DOR in trying to achieve their reproductive goals, but with a very low success rate if their own eggs are used.

In light of the limitations described above, the aim of the present invention is to provide a composition for use in improving female fertility.

Within the scope of this aim, an object of the invention is to provide a composition that makes it possible to increase the ovarian reserve.

Another object of the invention is to provide a composition that makes it possible to improve the quality of the ovarian reserve.

Yet another object of the invention is to provide a composition that allows improving the clinical outcomes of in vitro fertilization.

This aim, as well as these and other objects which will become more apparent hereinafter, are achieved by a composition comprising fenugreek for use in the treatment of female infertility.

The aim and objects of the invention are also achieved by a dietary supplement comprising the composition for the use of the invention.

Finally, the aim and objects of the invention are also achieved by a method for treating female infertility that comprises the administration of a composition comprising fenugreek or constituted by fenugreek.

Further characteristics and advantages of the invention will become more apparent from the detailed description that follows.

In a first aspect, the present invention relates to a composition comprising fenugreek for use in the treatment of female infertility.

Within the scope of the invention, the term "composition comprising fenugreek" comprises compositions essentially constituted by or constituted by fenugreek.

In one embodiment, the treatment improves natural female fertility.

In other embodiments, the treatment improves the clinical outcomes of in vitro fertilization.

The inventors of the present invention have surprisingly observed that the composition for use according to the invention increases ovarian reserve.

The inventors of the present invention have also surprisingly demonstrated that the composition for use according to the invention increases the number of antral follicles and AMH levels.

In particular, the inventors have demonstrated that the administration of 600-900 mg per day of fenugreek alone or associated with one or more of Tribulus terrestris, Withania somnifera, forskolin, and zinc results in a statistically significant increase in ovarian reserve.

The composition described herein may be contained in a healthcare product, for example a dietary supplement, a nutraceutical, a medical device, a drug, or a food for special medical purposes.

The composition according to the invention optionally also comprises one or more physiologically acceptable excipients. Any excipient suitable for the preparation of the desired dosage form for administration to humans is contemplated for use with the combination of active ingredients contained in the composition of the present invention.

For the purposes of the present application, the expression "physiologically acceptable" is understood to refer to edible substances that are approved by health authorities for use in pharmaceutical, phytotherapeutic, nutritional, or food applications. A physiologically acceptable excipient may be a pharmaceutically acceptable excipient.

The composition of the present invention comprises any composition provided by means of the active ingredient (fenugreek) or the active ingredients mentioned above (fenugreek and one or more of Tribulus terrestris, Withania somnifera, forskolin, and zinc) and optionally one or more physiologically acceptable excipients. These compositions are suitable for food, nutritional, or dietary use in a subject, wherein the subject is preferably a mammal, in particular a human being.

In a second aspect, the present invention therefore relates to a dietary supplement that comprises the composition of the invention described above.

The preparation of a dietary supplement based on fenugreek (Trigonella foenum-graecum) may vary according to the desired final form (capsules, powder, liquid extract, etc.) . The general process for obtaining a composition based on fenugreek or a supplement containing fenugreek comprises the following steps:

### 1. Seed harvesting and selection

The mature seeds of the fenugreek plant are harvested. The seeds are dried and cleaned to remove impurities.

### 2. Grinding

The dried seeds are finely ground to obtain a powder.

### 3. Extraction (optional)

In some cases, an extraction process using solvents (water or alcohol) is performed to concentrate the active ingredients, such as steroidal saponins (e.g., diosgenin), fibers, and phytosterols. The extract is then dried to obtain a concentrated powder.

### 4. Standardization (optional)

The powder or extract can be standardized to ensure a specific amount of active ingredient per dose (e.g., 50% saponins).

### 5. Formulation

The powder (pure or extract) is mixed with excipients (such as anti-agglomerating agents or fillers, such as magnesium stearate or microcrystalline cellulose).

### 6. Encapsulation or compression

The compound is encapsulated in vegetable or gelatin capsules, or compressed into tablets.

### 7. Packaging

The capsules or tablets are packaged in blister packs or bottles, labeled, and sealed.

The present invention also relates to a method for treating female infertility that comprises the administration of a composition comprising fenugreek or constituted by fenugreek.

Preferably, fenugreek is administered in an amount comprised between 600 and 900 mg per day in one or more doses.

In a preferred embodiment, the composition further comprises one or more of Tribulus terrestris, Withania somnifera, forskolin, and zinc.

In embodiments, fenugreek is administered in combination with one or more of:
- Coleus forskohlii extract standardized to 10% forskolin (500 mg per day);
- Withania somnifera extract standardized to 2.5% withanoids (600 mg per day);
- Tribulus terrestris extract (750 mg per day);
- zinc gluconate (74 mg per day), and
- folic acid (0.4 mg per day).

Preferably, the treatment method according to the invention is used to enhance reproductive capacity in women with reduced fertility due to diminished ovarian reserve.

The invention will now be further illustrated by the following nonlimiting example:

### EXAMPLE 1: EVALUATION OF THE EFFECT OF ADMINISTRATION OF FENUGREEK ON NATURAL FEMALE FERTILITY

A prospective study was conducted in which 80 women with diminished ovarian reserve were enrolled: 40 women in GROUP I (treatment) and 40 women in GROUP II (control), divided into:
GROUP Ia constituted by 20 women who twice daily for 60 days took one 450 mg tablet containing:
   - 300 mg of fenugreek standardized to 50% saponins;
   - 150 mg of excipients (including 100 mg of dibasic calcium phosphate (CaHPO₄), 25 mg of a mixture of mono-, di-, and triglycerides of behenic acid (marketed as "Compritol"), 15 mg of silicon dioxide, and 10 mg of magnesium salts of fatty acids,
GROUP Ib: 20 women who took the same fenugreek-based supplement as GROUP Ia for 60 days three times a day,
GROUP IIa: 20 women who were administered for 60 days twice a day a 450 mg placebo tablet constituted by 300 mg of dibasic calcium phosphate (CaHPO₄), 75 mg of a mixture of mono-, di-, and tri-glycerides of behenic acid (marketed as "Compritol"), 45 mg of silicon dioxide, and 30 mg of magnesium salts of fatty acids, and
GROUP IIb: 20 women who were administered three times daily for 60 days a 450 mg tablet of the same placebo as GROUP IIa.

Serum levels of AMH, FSH, estradiol, and antral follicle count were determined before and after fenugreek supplementation in each group.

### Statistical analysis

Data analysis was performed using Statistical Package for the Social Sciences version 15.0 (SPSS Inc., Chicago, IL, USA). The distributions of continuous and discrete variables, whether normal or not, were determined using the Shapiro-Wilk test, the t-test for paired samples, and the Wilcoxon signed-rank test. Data are presented as ± mean, standard deviation, and range.

### Results

Significant differences were found in the levels of FSH, estradiol, AFC, and AMH on the third day before and after supplementation of fenugreek in all patients (GROUP I) (p = 0.001, 0.001, 0.002, 0.001, and 0.001, respectively) compared to the control group (Table I).

**TABLE 1**

| GROUP I (overall mean of groups Ia and Ib) | Before treatment | After treatment | p-value |
|---|---|---|---|
| FSH (mIU/mL) | 13.73 ± 8.27 | 10.36 ± 6.18 | 0.001 |
| Estradiol (pg/mL) | 51.78 ± 12.48 | 35.25 ± 13.18 | 0.001 |
| Antral follicle count | 4.12 ± 0.87 | 4.37 ± 0.94 | 0.002 |
| AMH (ng/mL) | 0.32 ± 0.29 | 0.75 ± 0.70 | 0.001 |

| GROUP II (placebo) | Before treatment | After treatment | p-value |
|---|---|---|---|
| FSH (mIU/mL) | 13.71 ± 7.27 | 13.6 ± 7.21 | NS |
| Estradiol (pg/mL) | 53.48 ± 12.34 | 55.68 ± 12.78 | NS |
| Antral follicle count | 4.24 ± 0.77 | 4.21 ± 0.843 | NS |
| AMH (ng/mL) | 0.35 ± 0.398 | 0.356 ± 0.26 | NS |

| | | | |
|---|---|---|---|
| p < 0.05 is the commonly accepted threshold for declaring a statistically significant difference. p < 0.01 indicates a very significant difference. p < 0.001 indicates a highly significant difference. NS = not statistically significant | | | |

Statistically significant differences were found in AFC and AMH values between GROUP Ia and GROUP Ib (AFC 4.47 ± 0.77 vs 4.74 ± 0.87 p = 0.025; AMH 0.43 ± 0.31 vs 0.79 ± 0.52 p = 0.003) (Table II).

**TABLE II**

| | FENUGREEK 600 mg per day | FENUGREEK 900 mg per day | p-value |
|---|---|---|---|
| AFC | 4.47 ± 0.77 | 4.74 ± 0.87 | 0.025 |
| AMH | 0.43 ± 0.31 | 0.79 ± 0.52 | 0.003 |

Moreover, 8 spontaneous pregnancies occurred only in Group I.

Overall, as shown in Table III, Group I (mean of Ia and Ib) showed an increase in both the clinical pregnancy rate and the live birth rate compared to the placebo:

**TABLE III**

| | GROUP I | PLACEBO | p-value |
|---|---|---|---|
| Clinical pregnancy rate | 28.10% | 10.90% | < 0.01 |
| Live birth rate | 23.10% | 4.00% | < 0.05 |

### EXAMPLE 2: EVALUATION OF THE EFFECT OF FENUGREEK ADMINISTRATION ON FEMALE IN VITRO FERTILITY

An evaluation was also conducted to determine whether supplementation with fenugreek (300 mg twice daily) could improve clinical parameters in in vitro fertilization programs. A total of 33 women were enrolled, 17 in the study group (fenugreek) and 16 in the control group. No differences were found between the two groups with respect to general characteristics (age, AFC, AMH, BMI).

Controlled ovarian stimulation (COS) was performed for all patients using a short protocol with gonadotropin-releasing hormone antagonists (GnRh-a). Treatment with recombinant gonadotropins was initiated on the 2nd day of the menstrual cycle on the basis of the patient's characteristics. During COS, ovarian response was monitored by routine serum testing of levels of luteinizing hormone (LH) and estradiol (E2) and ultrasound measurement of follicular size every 2 or 3 days. Doses were adjusted on the basis of the patient's response. Ganirelix (Orgalutran 0.25 mg/50 ml, MSD, Italy) was administered starting on the day when a follicle measuring more than 13 mm was identified. When at least one follicle reached a diameter of ≥ 18 mm, recombinant human chorionic gonadotropin (rhCG, Ovitrelle, Merck Serono, Italy) or 0.3 mg of triptorelin acetate (Decapeptyl, Merck, Italy) was administered to induce ovulation; oocyte harvesting was performed 35-36 hours later under sedation. All oocytes included in the study were inseminated by means of intracytoplasmic sperm injection (ICSI). Clinical outcomes were evaluated for clinical pregnancy and live birth rates.

A comparison between the two groups after the first stimulation cycle revealed higher rates of clinical pregnancy (23.5% vs. 12.5%, p-value = 0.25) and live births among patients in the study group (17.6% vs. 6%, p-value = 0.20) (Table III).

The data demonstrate that the administration of fenugreek significantly increases both the AMH level and the antral follicle count, resulting in an improvement in natural fertility and the clinical outcome of in vitro fertilization.

The disclosures in Italian Patent Application No. 102025000011054 from which this application claims priority are incorporated herein by reference.

## Claims

1. A composition comprising fenugreek for use in the treatment of female infertility.

2. The composition for use according to claim 1, wherein said treatment improves natural fertility.

3. The composition for use according to claim 1, wherein said treatment improves the clinical results of in vitro fertilization, in particular clinical pregnancy and live birth rate.

4. The composition for use according to any one of the preceding claims, wherein said female infertility is caused by a reduction in ovarian reserve.

5. The composition for use according to any one of the preceding claims, wherein said treatment increases ovarian reserve.

6. The composition for use according to any one of the preceding claims, wherein said treatment increases anti-Müllerian hormone (AMH) levels.

7. The composition for use according to any one of the preceding claims, wherein said treatment increases the antral follicle count (AFC).

8. The composition for use according to any one of the preceding claims, wherein said treatment comprises the administration of fenugreek in an amount comprised between 600 and 900 mg per day.

9. The composition for use according to any one of the preceding claims, wherein said composition further comprises one or more of Tribulus terrestris, Withania somnifera, forskolin and zinc.

10. A dietary supplement comprising the composition for use of any of the preceding claims.
